# EUROPÄISCHE PATENTSCHRIFT

(11) **EP 2 869 868 B1**
(45) Veröffentlichungstag und Bekanntmachung des Hinweises auf die Patenterteilung: **13.04.2016**
(21) Anmeldenummer: 14718079.8
(22) Anmeldetag: 16.04.2014
(51) Int. Cl.: A61M 1/36, A61M 39/22

(54) **BEFÜLLVORRICHTUNG EINES FLÜSSIGKEITSSYSTEMS**
FILLING DEVICE OF A FLUID SYSTEM
DISPOSITIF DE REMPLISSAGE D'UN CIRCUIT DE LIQUIDE

(30) Priorität: 19.04.2013 DE 202013101695 U; 13.01.2014 DE 102014100324
(43) Veröffentlichungstag der Anmeldung: 13.05.2015
(73) Patentinhaber: B. Braun Avitum AG, 34212 Melsungen (DE)
(72) Erfinder: HUNDERTMARK, Charleen, 34119 Kassel (DE); HIERONYMI, Judith, 34281 Gudensberg (DE); SUSCA, Michele, 69221 Dossenheim (DE); MIHAILESCU, Anne-Marie, 34212 Melsungen (DE); FREITAG, Claudia, 34281 Gudensberg (DE); HECTOR, Rainer, 49082 Osnabrück (DE)
(74) Vertreter: Winter, Brandl, Fürniss, Hübner, Röss, Kaiser, Polte - Partnerschaft mbB
(86) Internationale Anmeldenummer: PCT/EP2014/057748
(87) Internationale Veröffentlichungsnummer: WO 2014/170381

(56) Entgegenhaltungen:
- US-A- 4 819 653
- US-A- 5 334 315
- US-A- 5 647 845
- US-A1- 2008 214 981

## Beschreibung

Die vorliegende Erfindung betrifft ein Verfahren zur Durchführung eines Befüll- und Rezirkulationsvorgangs eines blutseitigen Leitungssystems einer Dialysemaschine unter Verwendung einer Befüllvorrichtung.

### Hintergrund der Erfindung

Das hydraulische System (blut-seitiges Flüssigkeitssystem) eines Blutbehandlungsgeräts, beispielsweise einer Dialysemaschine muss vor Anschluss an einen Patienten mit einer Flüssigkeit, beispielsweise einer NaCl - Lösung oder einer anderen sterilen physiologischen Lösung, gefüllt werden, derart, dass Lufteinschlüsse in dem System entlüftet werden, die für einen, an das System flüssigkeits - angeschlossenen Patienten gefährlich sein würden. Des Weiteren kann das hydraulische System mit der eingefüllten Flüssigkeit über einen bestimmten Zeitraum durchgespült werden, um im System möglicherweise abgelagerte Schadstoffe, Schmutzpartikel, etc. auszufiltern/auszuschwemmen, bevor das System am Patienten angelegt wird. Diese beiden Vorgänge werden bei einem extrakorporalen Blutbehandlungsgerät im Rahmen eines Befüll - Zirkulations - Zyklus durchgeführt.

### Stand der Technik

Aus US 5,334,315 ist eine Befüllvorrichtung für ein Leitungssystem bekannt, bei der ein Spike zur Verbindung mit einem Fluidbehälter sowie eine Fluid-Sperreinrichtung vorgesehen sind.

Im Stand der Technik existieren Flüssigkeitsbehälter vorzugsweise in Form von Kunststoffbeuteln, die speziell für extrakorporale Blutbehandlungsgeräte dieser einschlägigen Gattung konstruiert sind, um die u. a. wie vorstehend definierten Gerätefunktionen zu ermöglichen. Solche Flüssigkeitsbehälter werden auch von der Anmelderin der vorliegenden Anmeldung hergestellt und vertrieben.

Ein solcher Flüssigkeitsbehälter hat in der Regel eine Flüssigkeitsaufnahmekammer und zwei vorzugsweise verschließbare Flüssigkeitsanschlüsse. An einen ersten der zwei Anschlüsse ist ein arterieller Leitungsabschnitt und an den zweiten Anschluss ein venöser Leitungsabschnitt des hydraulischen Systems (Fluidsystems oder auch als Fluidleitungssystem bezeichnet) des extrakorporalen Blutbehandlungsgeräts anschließbar. Der Flüssigkeitsbeutel sowie die beiden Leitungsabschnitte bilden zusammen eine Zirkulationsvorrichtung des extrakorporalen Blutbehandlungsgeräts.

Für den Fluidsystem - Befüllvorgang wird zunächst der arterielle Leitungsabschnitt an den ersten Flüssigkeitsanschluss des Beutels angeschlossen und nach Öffnen des ersten Flüssigkeitsanschlusses das hydraulische System befüllt. Dabei verbleibt der venöse Leitungsabschnitt des Systems zunächst zur Atmosphäre hin offen oder ist an einen Ablauf, ein Behältnis oder einen Beutel angeschlossen, sodass im System befindliche Luft zur Atmosphäre hin entweichen/entlüftet werden kann. Sobald der Befüllvorgang abgeschlossen ist, wird der venöse Leitungsabschnitt an den zweiten Flüssigkeitsanschluss des Beutels angeschlossen, um dann die im hydraulischen System des extrakorporalen Blutbehandlungsgeräts befindliche Flüssigkeit für eine bestimmte Zeitspanne oder um ein bestimmtes Strömungsvolumen über die Beutelkammer zu zirkulieren.

Bei diesem optionalen Zirkulationsvorgang durchströmt die Flüssigkeit systeminterne Filtereinrichtungen, in denen restliche Lufteinschlüsse mit der Flüssigkeit entfernt/ausgefiltert werden. Bei Bedarf kann der venöse Leitungsabschnitt des hydraulischen Systems wieder vom zweiten Flüssigkeitsanschluss des Fluidbeutels abgeklemmt und die im hydraulischen System befindliche Flüssigkeit unter ständiger Zufuhr von Flüssigkeit aus dem Behälter nochmals ausgespült werden.

Mit Beendigung des Zirkulationsvorgangs ist der eine Patientenbehandlung vorbereitende Befüll-/Zirkulationszyklus abgeschlossen, sodass die beiden Leitungsabschnitte (venös und arteriell) vom Flüssigkeitsbeutel abgetrennt und an den Patienten für eine Behandlung angelegt werden können.

Aus der vorstehenden Beschreibung des Befüll-/Zirkulationszyklus eines aus dem Stand der Technik bekannten hydraulischen Systems/Flüssigkeitsleitungssystems eines extrakorporalen Blutbehandlungsgeräts (Dialysemaschine) wird ersichtlich, dass für die Befüll- und Zirkulationsvorgänge der Fluidbeutel im Systemkreis verbleibt, d. h. die im System befindliche Flüssigkeit durch den Fluidbeutel bzw. durch dessen Fluidkammer zirkuliert wird. Damit kann möglicherweise die im Fluidbeutel bevorratete Flüssigkeit kontaminiert werden. Dies hat zur Folge, dass mit jeder neuen Behandlungsvorbereitung des extrakorporalen Blutbehandlungsgeräts ein neuer Fluidbeutel mit frischer, unkontaminierter Flüssigkeit für den folgenden Befüll-/Zirkulationszyklus eingesetzt wird, wohingegen der Fluidbeutel für den zuvor ausgeführten Befüll-/Zirkulationszyklus entsorgt wird, unabhängig von dessen Restfüllstand. Es liegt auf der Hand, dass bei dieser Vorgehensweise im Fall einer hohen Behandlungszahl ein mengenmäßig großer Teil an Flüssigkeit verschwendet wird, da der Flüssigkeitsinhalt eines Flüssigkeitsbeutels nur für einen Befüll-/Zirkulationszyklus (unvollständig) verwendbar ist.

Des Weiteren betreffen die Fluidbeutel für Blutbehandlungsgeräte quasi eine Sonderanfertigung mit zwei separaten Fluidanschlüssen, wodurch sich aufgrund der gegenüber konventioneller NaCl -Beutel/Flaschen kleinen Stückzahlen die Fertigung insgesamt verteuert.

Angesichts dieser Problematik ist es die Aufgabe der vorliegenden Erfindung, eine gattungsgemäße Befüllvorrichtung eines Fluidsystems vorzugsweise eines extrakorporalen Blutbehandlungsgeräts sowie ein Befüllverfahren bereit zu stellen, was gegenüber dem Stand der Technik wirtschaftlicher und damit kostengünstiger betrieben werden kann. Des Weiteren ist ein Ziel der vorliegenden Erfindung, ein mit der erfindungsgemäßen Befüllvorrichtung ausgerüstetes Fluidsystem beispielsweise eines extrakorporalen Blutbehandlungsgeräts bereit zu stellen, das in einfacher Weise bedient werden kann und die Verwendung konventioneller Fluidflaschen (z.B. NaCl-Flaschen) erlaubt.

Diese Aufgabe wird gemäß Anspruch 1 durch ein Verfahren zur Durchführung eines Befüll- und Rezirkulationsvorgangs eines blutseitigen Leitungssystems einer Dialysemaschine gelöst.

Vorteilhafte Ausgestaltungen der Erfindung sind Gegenstand der Unteransprüche.

Der Grundgedanke der vorliegenden Erfindung besteht darin, die gattungsgemäße Befüllvorrichtung eines Fluidleitungssystems insbesondere eines extrakorporalen Blutbehandlungsgeräts so auszubilden, dass ein konventioneller (preisgünstiger) Fluidbehälter bekannter Bauart, etwa eine herkömmliche NaCl-Flasche oder ein entsprechender Beutel mit einem einzigen punktierbaren/ konnektierbaren Verschluss verwendet werden kann. Demzufolge hat die erfindungsgemäße Befüllvorrichtung als zentrale Bauelemente einen sogenannten Spike für das Konnektieren des einzigen Fluidanschlusses eines konventionellen medizinischen Fluidbehälters (z.B. NaCl-Flasche), an den sich stromab eine manuell betätigbare Fluid-Sperreinrichtung, etwa ein Sperrventil anschließt, die dazu angepasst oder dafür vorgesehen ist, fortdauernd mit dem Spike fluidverbunden zu bleiben. Die Fluid-Sperreinrichtung hat zumindest einen Fluid-Auslassanschluss, der dafür angepasst ist, dass ein Leitungsabschnitt/Schlauch eines Fluidleitungssystems/Flüssigkeitssystems vorzugsweise der arterielle Leitungsabschnitt eines Blutreinigungsgeräts/Dialysemaschine daran lösbar anschließbar ist.

Durch die Anordnung der Fluid-Sperreinrichtung am Spike kann der Spike an dem konventionellen medizinischen Fluidbehälter nach Punktieren des Behälterverschlusses verbleiben, wohingegen der arterielle Leitungsabschnitt wahlweise an die Fluid-Sperreinrichtung für einen Befüllvorgang des Fluidleitungssystems anschließbar und danach wieder von der Fluid-Sperreinrichtung abklemmbar ist, ohne dass Fluid aus dem medizinischen Fluidbehälter verloren geht. Da der arterielle Leitungsabschnitt von dem Auslassanschluss der Fluid-Sperreinrichtung (z.B. Luer-Lock-Anschluss) abgekoppelt wird, kann der arterielle Leitungsabschnitt unmittelbar danach an einen arteriellen Patientenzugang wieder angeschossen werden, ohne dass am arteriellen Leitungsabschnitt bzw. dessen Anschluss irgend welche Veränderungen vorgenommen werden müssen. Dadurch wird die Handhabung der Befüllvorrichtung vereinfacht.

Als Fluid-Sperreinrichtung ist eine 3 - Wege - Weiche, beispielsweise ein Y- oder T-Stück oder ein 3 - Wege - Hahn, vorgesehen,
wobei die Wege/Anschlüsse wahlweise absperrbar sind (beispielsweise über Klemmen an den Wegen/Anschlüssen oder über den Hahn), um so die Weiche vollständig zu verschließen und/oder die Wege wahlweise miteinander Fluid zu koppeln bzw. eine Fluidströmung zumindest zwischen zwei ausgewählten Wegen zuzulassen. Erfindungsgemäß ist die 3 - Wege - Weiche, vorzugsweise der 3 - Wege - Hahn unmittelbar diesen einem vorzugsweise universellen medizinischen Flüssigkeitsbehälter nachgeordnet. Im Konkreten wird ein erster Anschluss der 3 - Wege - Weiche (des 3 - Wege - Hahns) mit dem sogenannten Spike (oder einem andersartigen Anschlussmittel) gekoppelt oder ist mit diesem ausgebildet, mittels dem eine Fluidkammer des Flüssigkeitsbehälters angezapft werden kann. An einen zweiten Anschluss der 3 - Wege - Weiche (des 3 - Wege - Hahns) ist der arterielle Leitungsabschnitt und an einen dritten Anschluss der 3 - Wege - Weiche (des 3 - Wege - Hahns) ist ein venöser Leitungsabschnitt des Fluidsystems anschließbar. Die 3 - Wege - Weiche, vorzugsweise der 3 - Wege - Hahn ist weiter vorzugsweise manuell in wenigstens drei Stellungen bringbar bzw. schaltbar, von denen in einer ersten Schaltstellung der erste Anschluss ausschließlich mit dem zweiten Anschluss fluidverbunden bzw. eine Fluidströmung zwischen diesen beiden Anschlüssen zugelassen ist und der dritte Anschluss gesperrt ist (single - Pass - Schaltstellung), in einer zweiten Schaltstellung der zweite Anschluss mit dem dritten Anschluss fluidverbunden bzw. eine Fluidströmung zwischen diesen beiden Anschlüssen zugelassen ist und der erste Anschluss gesperrt ist (Zirkulation - Schaltstellung) und in einer dritten Schaltstellung alle drei Anschlüsse voneinander getrennt bzw. gesperrt sind.

Durch ein derart ausgerüstetes medizinisches Fluid-/Flüssigkeitssystem ist es möglich, den herkömmlichen Flüssigkeitsbehälter für den Befüllvorgang an den arteriellen Leitungsabschnitt zu koppeln (bei unangeschlossenem venösen Leitungsabschnitt), indem die 3 - Wege - Weiche, vorzugsweise der 3 - Wege - Hahn in die erste Schaltstellung gebracht wird, und den arteriellen Leitungsabschnitt mit dem venösen Leitungsabschnitt für den Zirkulationsvorgang zu verbinden, indem die 3 - Wege - Weiche, vorzugsweise der 3 - Wege - Hahn in die zweiten Schaltstellung gebracht wird. Da in dieser zweiten Schaltstellung der erste Anschluss der 3 - Wege - Weiche, vorzugsweise des 3 - Wege - Hahns gesperrt ist, wird der Flüssigkeitsbehälter vom hydraulischen System/Kreis des extrakorporalen Blutbehandlungsgeräts getrennt, sodass die darin sich befindende Restflüssigkeit nicht von der im hydraulischen System zirkulierenden Flüssigkeit kontaminiert wird. Da in der dritten Schaltstellung der 3 - Wege - Weiche, vorzugsweise des 3 - Wege - Hahns dessen sämtliche Anschlüsse gesperrt sind, können in dieser Schaltstellung die beiden Leitungsabschnitte des hydraulischen Systems abgeklemmt und an den Patienten für eine Behandlung angelegt werden.

Der Flüssigkeitsbehälter kann somit für mehrere aufeinanderfolgende Behandlungen eingesetzt werden in Abhängigkeit von dessen Füllvolumens, sodass keine Flüssigkeit verloren geht. Des Weiteren ist die medizinische Befüllvorrichtung für einen konventionellen/universellen medizinischen Flüssigkeitsbehälter vorgesehen, der gegenüber den für extrakorporale Blutbehandlungsgeräte speziell angefertigten Behältern mit zwei Anschlüssen preisgünstiger ist. Schließlich braucht der verwendete Flüssigkeitsbehälter gar keinen Anschluss insbesondere für den Fall, dass an die 3 - Wege - Weiche, vorzugsweise den 3 - Wege - Hahn der sogenannte Spike angeschlossen oder mit diesem zu einer integralen Baueinheit zusammengefasst ist. Im letzteren Fall kann der Spike quasi übergangslos, d.h. ohne Zwischenschaltung eines (überbrückenden) Leitungsstücks unmittelbar mit dem ersten Anschluss vorzugsweise einstückig verbunden oder mit diesem verschraubt sein.

Das medizinische Fluidsystem/Fluidleitungssystem vorzugsweise eines Blutreinigungsgeräts (Dialysemaschine) hat eine Befüllvorrichtung gemäß vorstehend beschriebenem Aufbau sowie einen venösen und einen arteriellen Leitungsabschnitt, von denen zumindest der arterielle Leitungsabschnitt dafür angepasst ist, wahlweise an die Befüllvorrichtung angeschlossen zu sein/zu werden. Des Weiteren hat zumindest der arterielle Leitungsabschnitt unmittelbar stromab zu seinem mit der Befüllvorrichtung zusammenwirkenden Anschluss (ein Bauelement) eine Sperreinrichtung (z.B. Schlauchklemme als weiteres Bauelement)), mittels welcher der arterielle Leitungsabschnitt temporär für ein Umklemmen von der Befülleinrichtung zu einem arteriellen Patientenzugang und umgekehrt fluidgesperrt werden kann. Vorzugsweise weist auch der venöse Leitungsabschnitt die gleichen Bauelemente auf wie der arterielle Leitungsabschnitt.

Die Erfindung wird nachstehend anhand eines bevorzugten Ausführungsbeispiels unter Bezugnahme auf die begleitenden Figuren näher erläutert.
Fig. 1 zeigt ein Fluidsystem vorzugsweise in Form einer Zirkulationsvorrichtung eines extrakorporalen Blutbehandlungsgeräts gemäß einem bevorzugten Ausführungsbeispiel der Erfindung,
Fig. 2 zeigt eine geschlossene Schaltstellung einer Befüllvorrichtung in Form eines 3 - Wege - Hahns der Zirkulationsvorrichtung gemäß Fig. 1 als eine mögliche Variante der Befüllvorrichtung (hier 3 - Wege - Weiche), wobei an dieser Stelle bereits darauf hingewiesen wird, dass beispielsweise auch ein Y- oder T-Stück mit Schlauchsperrmittel (Schlauchklemmen) an jedem Zweigweg stromauf zu den Befüllvorrichtungs-internen Anschlüssen vorgesehen sein kann,
Fig. 3 zeigt eine "single-pass" Schaltstellung des 3 - Wege - Hahns der Zirkulationsvorrichtung gemäß Fig. 1, in der eine Flüssigkeitsquelle (Flüssigkeitsbehälter) ausschließlich mit einem arteriellen Leitungsabschnitt des extrakorporalen Blutbehandlungsgeräts fluidverbunden ist,
Fig. 4 zeigt eine "Zirkulation" Schaltstellung des 3 - Wege - Hahns der Zirkulationsvorrichtung gemäß Fig. 1, in welcher der arterielle Leitungsabschnitt ausschließlich mit einem venösen Leitungsabschnitt des extrakorporalen Blutbehandlungsgeräts fluidverbunden (kurzgeschlossen) ist und die Flüssigkeitsquelle vom extrakorporalen Blutbehandlungsgerät fluidgetrennt ist und
Fig. 5 zeigt eine andere geschlossene Schaltstellung eines 3 - Wege - Hahns der Zirkulationsvorrichtung gemäß Fig. 1.

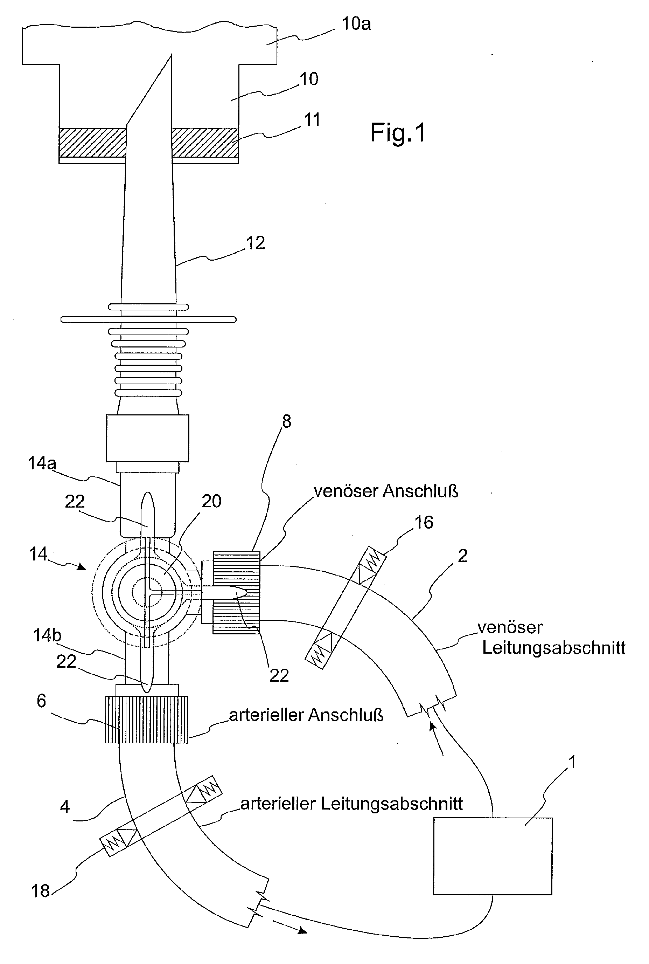

Gemäß der Fig. 1 hat ein extrakorporales Blutbehandlungsgerät 1, beispielsweise eine Dialyse- oder Apheresemaschine ein internes hydraulisches Leitungssystem (nachfolgend als Fluidleitungssystem bezeichnet), durch das während einer Behandlungsphase auf der Maschinenseite beispielsweise eine Blut - Reinigungsflüsssigkeit (Dialysierflüssigkeit) geleitet wird und auf der Patientenseite Blut extrakorporal hindurchfließt, wobei das maschinenseitige und patientenseitige Fluidleitungssystem im Fall einer Dialysemaschine durch einen nicht weiter dargestellten Dialysator (Filter) fluidisch getrennt sind.

Hierfür hat das Fluidleitungssystem patientenseitig einen venösen sowie einen arteriellen Leitungsabschnitt 2, 4, vorzugsweise jeweils Schlauchabschnitt mit jeweils endseitig angeordneten/ ausgebildeten Anschlüssen (Luer-Lock-Anschlüssen) 6, 8, an die beispielsweise Injektionsnadeln oder Kanülen (nicht dargestellt) als Patientenzugang anschließbar sind, welche in einen Patientenkörper einführbar sind.

Um ein möglicherweise notwendiges Ausschwemmen von ggf. herstellungsbedingten Verunreinigungen in den Patientenkörper zu vermeiden, hat das extrakorporale Blutbehandlungsgerät 1 eine Befüllvorrichtung , welche vorliegend einen Zirkulationsprozess ermöglicht (daher nachfolgend als Zirkulationsvorrichtung bezeichnet), mittels welcher das Fluidleitungssystem in der Regel vor jeder Patientenbehandlung gereinigt wird.

Gemäß der Fig. 1 hat die Befüll-/Zirkulationsvorrichtung eine Flüssigkeitsquelle vorzugsweise in Form eines universellen Flüssigkeitsbehälters (NaCl-Flasche) 10 mit einem einzigen Auslass 11, der beim vorliegenden Ausführungsbeispiel mittels eines Spike 12 der Befüllvorrichtung punktiert wird, um Flüssigkeit aus dem Flüssigkeitsbehälter 10 abzuzapfen. Der Aufbau des Spike 12 entspricht bekannter Spikekonstruktionen, sodass an dieser Stelle auf dessen Aufbau nicht weiter eingegangen werden muss. Ferner sei darauf hingewiesen, dass beispielsweise im Fall eines Luer-Lock- oder andersartigen Anschlusses auf der Behälterseite der Spike durch ein entsprechendes Anschlussstück auf Seiten der Befüll-/Zirkulationsvorrichtung ersetzt sein kann.

Des Weiteren hat die Befüll-/Zirkulationsvorrichtung eine Fluidsperreinrichtung vorliegend in Form einer 3 - Wege - Weiche, vorzugsweise einen 3 - Wege - Hahn 14, der dem Spike 12 (Anschlussstück) in Strömungsrichtung weg vom Flüssigkeitsbehälter 10 gesehen, unmittelbar nachgeschaltet ist. Im vorliegenden Fall ist der Spike 12 direkt (ohne Zwischenschaltung eines zusätzlichen Leitungsstücks) an den 3 - Wege - Hahn 14 angeschlossen. Alternativ kann der Spike 12 auch einstückig bzw. als eine Baueinheit mit dem 3 - Wege - Hahn 14 ausgebildet sein.

Der 3 - Wege - Hahn 14 hat hierfür einen ersten Anschluss oder Fluideinlass 14a, der mit dem Spike 12 fluidverbunden ist, bzw. an den die Flüssigkeitsquelle 10 anschließbar/angeschlossen ist. Des Weiteren hat der 3 - Wege - Hahn 14 einen zweiten Anschluss 14b, an den der arterielle Leitungsabschnitt 4 des patientenseitigen Fluidleitungssystems des extrakorporalen Blutbehandlungsgeräts 1 anschließbar ist. Schließlich hat der 3 - Wege - Hahn 14 einen dritten Anschluss 14c, an den der venöse Leitungsabschnitt 2 des patientenseitigen Fluidleitungssystems des extrakorporalen Blutbehandlungsgeräts 1 anschließbar ist. Sowohl der venöse wie auch der arterielle Leitungsabschnitt 2, 4 ist jeweils mit einer Schlauchklemme 16, 18 oder dergleichen Sperrmittel versehen, um den zugehörigen Schlauchabschnitt wahlweise temporär zu verschließen.

Der 3 - Wege - Hahn 14 hat vorliegend einen manuell betätigbaren Drehverschluss bestehend aus einem drehbaren zylindrischen Ventilkolben 20, der stirnseitig mit einer Handhabe vorzugsweise in Form von (drei) Eingriffsflügeln 22 versehen/ausgebildet ist. Der Ventilkolben hat eine zentrale Längsbohrung von der drei in Umfangsrichtung gleichmäßig beabstandete Radialbohrungen abzweigen. Die Eingriffsflügel 22 sind so angeordnet, dass sie sich längs der Radialbohrungen ausrichten und so die Strömungsrichtung der Radialbohrungen anzeigen. Ein solcher 3 - Wege - Hahn ist aus dem Stand der Technik hinlänglich bekannt, sodass an dieser Stelle auf eine weitere Beschreibung insbesondere von dessen Funktion verzichtet werden kann.

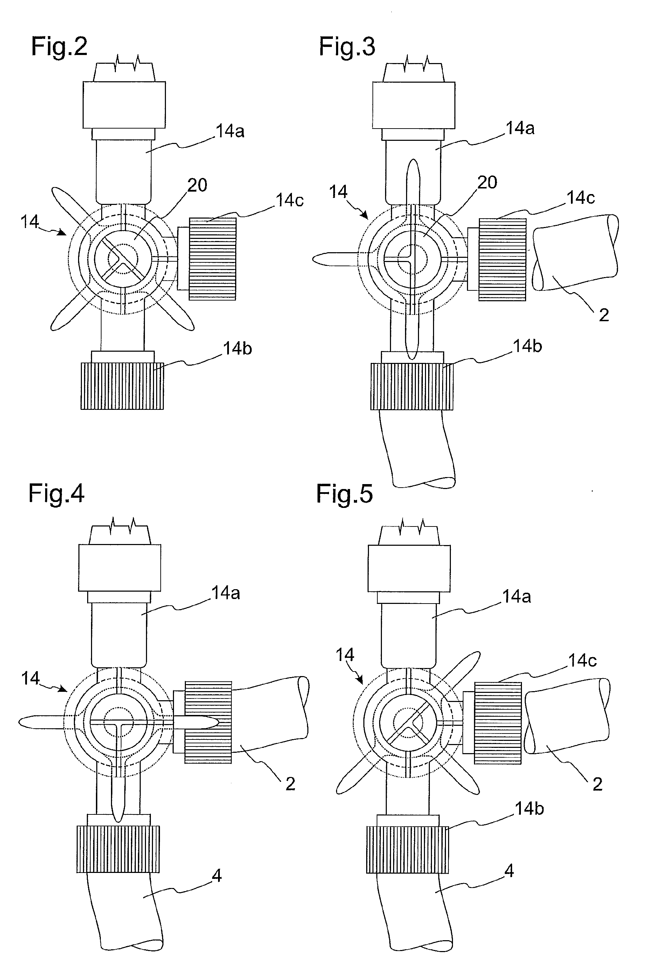

In den Fig. 2 bis 5 sind die erfindungsgemäß beabsichtigten Schaltstellungen des 3 - Wege - Hahns 14 in Abhängigkeit von den aktuellen Betriebsphasen des extrakorporalen Blutbehandlungsgeräts 1 dargestellt, welche nachfolgend in Zusammenhang mit den dadurch bezweckten Funktionen beschrieben werden.

Gemäß der Fig. 2 ist der 3 - Wege - Hahn 14 in einer Sperrposition gezeigt, in der alle drei Anschlüsse 14a - 14c geschlossen sind. In dieser Schaltstellung kann der Spike 12 den Auslass 12 des Universal - Flüssigkeitsbehälters 10 punktieren und somit die darin (in der vom Behälter ausgebildeten Kammer 10a) gespeicherte Flüssigkeit anzapfen, ohne dass Flüssigkeit zur Atmosphäre hin verloren geht.

Die Fig. 3 zeigt die so genannte "single pass" Schaltstellung, in welcher der erste Anschluss 14a mit dem zweiten Anschluss 14b fluidverbunden ist, wohingegen der dritte Anschluss 14c gesperrt wird. In dieser Schaltstellung ist bereits der arterielle Leitungsabschnitt 4 an den zweiten Anschluss 14b angeschlossen, wobei jedoch der venöse Leitungsabschnitt 2 zur Atmosphäre hin offen ist oder an einen Abfluss/Sammelbehälter angeschlossen ist.

In dieser Schaltposition wird Flüssigkeit (NaCl-Lösung) aus dem konventionellen Flüssigkeitsbehälter mit einem einzigen Anschluss über den 3 - Wege - Hahn 14 in den arteriellen Leitungsabschnitt 4 geleitet und dadurch das patientenseitige Fluidleitungssystem fortlaufend geflutet, solange, bis die Flüssigkeit aus dem venösen Leitungsabschnitt 2 heraus läuft. D.h. während dieses System-Füllvorgangs dient der venöse Leitungsabschnitt 2 als Entlüftung. Dabei sei darauf hingewiesen, dass während dieses Vorgangs die Schlauchklemmen 16, 18 natürlich geöffnet sind.

Sobald das System mit Flüssigkeit aus dem Flüssigkeitsbehälter 10 aufgefüllt ist, wird der venöse Leitungsabschnitt an den dritten Anschluss 14c des 3 - Wege - Hahns 14 angeschlossen und der 3 - Wege - Hahn 14 in die Schaltstellung gemäß der Fig. 4 verstellt, welche als "Zirkulation" Schaltstellung bezeichnet werden kann. In dieser Schaltstellung sind der zweite und der dritte Anschluss 14b, 14c des 3 - Wege - Hahns 14 miteinander fluidverbunden, wohingegen der erste Anschluss 14a gesperrt wird.

Wird nunmehr die im Fluidleitungssystem befindliche Flüssigkeit zirkuliert, durchströmt diese ausgehend vom venösen Leitungsabschnitt 2 den 3 - Wege - Hahn 14 und wird von dort wieder in den arteriellen Leitungsabschnitt 4 weiter geleitet, ohne dass Flüssigkeit in den Flüssigkeitsbehälter 10 eindringen kann. Die darin gespeicherte Flüssigkeit bleibt somit unkontaminiert.

Nach einer vorbestimmten Zirkulationsdauer wird der 3 - Wege - Hahn 14 in die in Fig. 5 gezeigte Schaltstellung weitergeschaltet, in welcher erneut alle drei Anschlüsse 14a - 14c gesperrt sind. Es ist zu erkennen, dass sich die Schaltstellung gemäß der Fig. 5 von der Schaltstellung gemäß der

Fig. 2 unterscheidet, da der Ventilkolben 20 nicht einfach in die erste Sperrstellung gemäß Fig. 2 voll - rückgedreht wurde, sondern in die zweite Sperrstellung gemäß der Fig. 5 teil - rückgedreht wurde, welche demnach der ersten Sperrstellung diametral gegenüberliegt. Wäre der Ventilkolben 20 nämlich voll - rückgedreht worden, hätte er zumindest die "single pass" Schaltstellung temporär durchlaufen, in der möglicherweise kontaminierte Flüssigkeit aus dem Fluidleitungssystem in den Flüssigkeitsbehälter hätte eindringen können.

Sobald der 3 - Wege - Hahn 14 gesperrt ist, wird nunmehr der venöse Leitungsabschnitt 2 erneut vom 3 - Wege - Hahn 14 abgeklemmt und der Ventilkolben 20 in die "single - pass" Stellung gemäß Fig. 3 gedreht, um die möglicherweise kontaminierte Flüssigkeit aus dem Fluidleitungssystem zu spülen. Mit Beendigung dieses Vorgangs ist der Befüll-/Zirkulationszyklus abgeschlossen.

Abschließend wird der 3 - Wege - Hahn 14 erneut gesperrt, alle Schlauchklemmen 16, 18 stromab zum 3 - Wege - Hahn 14 in Sperrstellung gebracht und auch der arterielle Leitungsabschnitt vom 3 - Wege - Hahn 14 abgeklemmt, um zusammen mit dem venösen Leitungsabschnitt am Patientenkörper angelegt werden zu können.

An dieser Stelle sei darauf hingewiesen, dass gemäß der vorstehenden Beschreibung die Befüllvorrichtung mit der 3 - Wege - Weiche als Fluid - Sperrvorrichtung ausgebildet ist, um einen Zirkulationsmodus ausführen zu können. Es ist aber auch denkbar, die Sperrvorrichtung als einfaches Schließventil mit einem Ein- und einem Auslassanschluss auszubilden, welches dann nur einen "single - pass - Betrieb" gemäß vorstehender Definition zulässt. In jedem Fall erlaubt die Befüllvorrichtung gemäß der vorliegenden Erfindung ein vorzugsweise manuelles Sperren das Spike - Auslasses, sodass der Spike nach Abschluss des "single - pass - Betriebs im Flüssigkeitsbehälter verbleiben und zumindest der hiervon abgeklemmte arterielle Leitungsabschnitt über dessen internen Anschluss an den bereits gelegten Patientenzugang ohne weitere Maßnahme angeschlossen werden kann.

Ferner ist es denkbar, eine weitere (oder alternativ zur voll geschlossenen Schaltposition) Schaltposition für die 3 - Wege - Weiche vorzusehen, in der alle drei Wege geöffnet und damit miteinander fluidverbunden sind. Diese Position hat dann technische Relevanz, wenn beispielsweise sterile Produkte verwendet werden.

Die vorliegende Erfindung betrifft zusammenfassend die Verwendung einer Befüllvorrichtung beilspielsweise in Form einer Zirkulationsvorrichtung eines extrakorporalen Blutbehandlungsgeräts 1 mit einem vorzugsweise universellen medizinischen Flüssigkeitsbehälter 10, an den wahlweise ein arterieller Leitungsabschnitt 4 eines Fluidleitungssystems des extrakorporalen Blutbehandlungsgeräts 1 anschließbar ist. Die Zirkulationsvorrichtung hat ferner einen Spike sowie eine Fluid-Sperrvorrichtung beispielsweise eine 3 - Wege - Weiche, vorzugsweise einen 3 - Wege - Hahn 14, der dem Spike 12 unmittelbar nachgeordnet ist. Ein Weg 14a der 3 - Wege - Weiche ist mit dem Spike 12 oder dergleichen Anschlussmittel gekoppelt oder einstückig mit diesem ausgebildet.

## Patentansprüche

1. Verfahren zur Durchführung eines Befüll- und Rezirkulationsvorgangs eines blutseitigen Leitungssystems einer Dialysemaschine unter Verwendung einer Befüllvorrichtung die folgende Bauelemente hat:
- einen Spike (12), der für das Konnektieren des einzigen Fluidanschlusses eines medizinischen Fluidbehälters (10) des Fluidsystems angepasst ist und
- eine manuell betätigbare Fluid-Sperreinrichtung (14), die unmittelbar stromab zu dem Spike (12) angeordnet und dazu angepasst ist, im Betrieb der Befüllvorrichtung mit dem Spike (12) fluidverbunden zu bleiben, wobei
- die Fluid-Sperreinrichtung (14) zumindest einen Fluidauslassanschluss in Form eines zweiten Weges (14b) hat, der dafür angepasst ist, dass ein arterieller Leitungsabschnitt eines blutseitigen Leitungssystems im Betrieb der Befüllvorrichtung daran lösbar anschließbar ist und
- die Fluid-Sperreinrichtung (14) eine 3 - Wege - Weiche, vorzugsweise ein 3 - Wege - Hahn (14) ist, welche dem Spike (12) unmittelbar nachgeordnet ist, **gekennzeichnet durch** die Schritte:
a. Anschließen des Spike (12) an den einzigen Fluidanschluss des medizinischen Fluidbehälters (10) bei vollständig gesperrter 3-Wege-Weiche (14) sowie Anschließen des arteriellen Leitungsabschnitts (4) an den zweiten Weg (14b) der noch gesperrten-3-Wege-Weiche (14) bei einem zur Umgebung hin geöffneten venösen Fluidleitungsabschnitt (2),
b. Durchspülen eines Fluidströmungspfads entlang des arteriellen Fluidleitungsabschnitts (4) und des venösen Fluidleitungsabschnitts (2) **durch** Fluidverbinden des Spike (12) mit dem zweiten Weg (14b) der 3-Wege-Weiche (14),
c. vollständiges Sperren der 3-Wege-Weiche (14) und Anschließen des venösen Fluidleitungsabschnitts (2) an einen dritten Weg (14c) der 3-Wege-Weiche (14),
d. Rezirkulieren des eingefüllten Fluids in dem Fluidströmungspfad **durch**
Kurzschließen des zweiten und dritten Wegs (14b, 14c) an der 3-Wege-Weiche bei gleichzeitigem Sperren des Spike (12),
e. vollständiges Sperren der 3-Wege-Weiche (14) und Abkoppeln des venösen Fluidleitungsabschnitts (2) vom dritten Weg (14c) sowie erneutes Durchspülen gemäß Schritt b. und
f. vollständiges Sperren der 3-Wege-Weiche als Vorbereitung für das darauf folgende Anschließen der befüllten arteriellen und venösen Fluidleitungsabschnitte (2, 4) an einen Patienten.

2. Verfahren zur Durchführung eines Befüll- und Rezirkulationsvorgangs eines blutseitigen Leitungssystems einer Dialysemaschine nach Anspruch 1, **dadurch gekennzeichnet, dass** ein erster Weg (14a) der 3 - Wege - Weiche, vorzugsweise des 3 - Wege - Hahns (14) den Fluideinlass bildet, der mit dem Spike (12) gekoppelt oder als Spike (12) ausgebildet ist, wobei an den zweiten Weg (14b) der 3 - Wege - Weiche, vorzugsweise des 3 - Wege - Hahns (14), welcher den zumindest einen Auslassanschluss bildet, der arterielle Leitungsabschnitt (4) und an einen dritten Weg (14c) der 3 - Wege - Weiche, vorzugsweise des 3 - Wege - Hahns (14) der venöse Leitungsabschnitt (2) des Fluidleitungssystems des extrakorporalen Blutbehandlungsgeräts (1) anschließbar sind.

3. Verfahren zur Durchführung eines Befüll- und Rezirkulationsvorgangs eines blutseitigen Leitungssystems einer Dialysemaschine nach Anspruch 2, **dadurch gekennzeichnet, dass** die 3 - Wege - Weiche, vorzugsweise der 3 - Wege - Hahn (14) in wenigstens drei Stellungen bringbar oder schaltbar ist, von denen in einer ersten Schaltstellung der erste Weg (14a) mit dem zweiten Weg (14b) fluidverbunden ist oder dazwischen eine Fluidströmung zulässt, und der dritte Weg (14c) gesperrt ist, in einer zweiten Schaltstellung der zweite Weg (14b) mit dem dritten Weg (14c) fluidverbunden oder eine Fluidströmung dazwischen zulässt und der erste Weg (14a) gesperrt ist und in einer dritten Schaltstellung alle drei Wege (14a-14c) gesperrt sind.

4. Verfahren zur Durchführung eines Befüll- und Rezirkulationsvorgangs eines blutseitigen Leitungssystems einer Dialysemaschine nach Anspruch 3, **dadurch gekennzeichnet, dass** der 3 - Wege - Hahn (14) manuell betätigbar ist, wofür dieser einen mit einer Handhabe (22) ausgebildeten Drehkolben (20) aufweist.

5. Verfahren zur Durchführung eines Befüll- und Rezirkulationsvorgangs eines blutseitigen Leitungssystems einer Dialysemaschine nach Anspruch 3 oder 4, **dadurch gekennzeichnet, dass** der Fluid- oder Flüssigkeitsbehälter (10) für den Befüllvorgang nach dem single-pass-Prinzip an den arteriellen Leitungsabschnitt (4) gekoppelt ist, indem die 3 - Wege - Weiche, vorzugsweise der 3 - Wege - Hahn (14) der Befüllvorrichtung in die erste Schaltstellung gebracht wird, und der arterielle Leitungsabschnitt (4) für den Zirkulationsvorgang mit dem venösen Leitungsabschnitt (2) verbunden ist, indem die 3 - Wege - Weiche, vorzugsweise der 3 - Wege - Hahn (14) in die zweite Schaltstellung gebracht wird, wobei in dieser zweiten Schaltstellung der erste Weg (14a) der 3 - Wege - Weiche, vorzugsweise des 3 - Wege - Hahns (14) gesperrt ist und damit der Flüssigkeitsbehälter (10) vom Fluidleitungssystem des extrakorporalen Blutbehandlungsgeräts (10) fluidgetrennt ist.

6. Verfahren zur Durchführung eines Befüll- und Rezirkulationsvorgangs eines blutseitigen Leitungssystems einer Dialysemaschine nach Anspruch 5, **dadurch gekennzeichnet, dass** in der dritten Schaltstellung der 3 - Wege - Weiche, vorzugsweise des 3 - Wege - Hahns (14) dessen sämtliche Wege (14a - 14c) gesperrt sind, sodass in dieser Schaltstellung die beiden Leitungsabschnitte (2, 4) des Fluidleitungssystems von dem Flüssigkeitsbehälter (10) abklemmbar sind.

7. Verfahren zur Durchführung eines Befüll- und Rezirkulationsvorgangs eines blutseitigen Leitungssystems einer Dialysemaschine nach einem der vorstehenden Ansprüche, **dadurch gekennzeichnet, dass** an die Fluid-Sperrvorrichtung (14) der Spike als Anschlussmittel (12) über einen Luer-Lock-Anschluss angeschlossen und/oder mit der Fluid-Sperrvorrichtung (14) zu einer integralen Baueinheit zusammengefasst ist.

8. Verfahren zur Durchführung eines Befüll- und Rezirkulationsvorgangs eines blutseitigen Leitungssystems einer Dialysemaschine nach Anspruch 7, **dadurch gekennzeichnet, dass** der Spike (12) übergangslos ohne Zwischenschaltung eines überbrückenden Leitungsstücks unmittelbar mit der Fluid-Sperrvorrichtung (14) verbunden ist.

## Claims

1. Method for performing a filling and recirculation process of a blood-side conducting system of a dialysis machine using a filling device which has the following components:
- a spike (12) which is adapted for connecting the single fluid connector of a medical fluid container (10) of the fluid system and
- a manually operable fluid blocking mechanism (14) which is arranged directly downstream of the spike (12) and is adapted to remain fluidically connected with the spike (12) while the filling device is in operation, whereby
- the fluid blocking mechanism (14) has at least one fluid outlet connector in the form of a second conduit (14b) which is adapted so that an arterial line section of a blood-side conducting system, can be connected to it in a detachable manner while the filling device is in operation
- the fluid blocking mechanism (14) is a 3-way switch, preferably a 3-way valve (14), which is arranged directly downstream of the spike (12), **characterized by** the steps:
a. Connecting of the spike (12) to the single fluid connector of the medical fluid container (10) with the 3-way switch (14) completely closed, and connecting of the arterial line section (4) to the second conduit (14b) of the still closed 3-way switch (14) with the venous fluid line section (2) open to the environment,
b. Flushing of a fluid flow path along the arterial fluid line section (4) and the venous fluid line section (2) by fluidically connecting the spike (12) with the second conduit (14b) of the 3-way switch (14),
c. Complete closing of the 3-way switch (14) and connecting of the venous fluid line section (2) to a third conduit (14c) of the 3-way switch (14),
d. Recirculating of the filled-in fluid in the fluid flow path by short-circuiting the second and third conduits (14b, 14c) on the 3-way switch with simultaneous closing of the spike (12),
e. Complete closing of the 3-way switch (14) and uncoupling of the venous fluid line section (2) from the third conduit (14c) and repeated flushing according to step b. and
f. Complete closing of the 3-way switch in preparation for the subsequent connecting of the filled arterial and venous fluid line sections (2, 4) to a patient.

2. Method for performing a filling and recirculation process of a blood-side conducting system of a dialysis machine according to claim 1, **characterised in that** a first conduit (14a) of the 3-way switch, preferably the 3-way valve (14), constitutes the fluid inlet which is coupled with the spike (12) or is designed as a spike (12), whereby on the second conduit (14b) of the 3-way switch, preferably the 3-way valve (14), which constitutes the at least one outlet connector, the arterial line section (4), and on a third conduit (14c) of the 3-way switch, preferably the 3-way valve (14), the venous line section (2) of the fluid conducting system of the extracorporeal blood treatment device (1) can be connected.

3. Method for performing a filling and recirculation process of a blood-side conducting system of a dialysis machine according to claim 2, **characterised in that** the 3-way switch, preferably the 3-way valve (14), can be put or switched in at least three positions, of which in a first switch position, the first conduit (14a) is fluidically connected with the second conduit (14b) or allows a fluid flow in between, and the third conduit (14c) is closed, in a second switch position, the second conduit (14b) is fluidically connected with the third conduit (14c) or allows a fluid flow in between, and the first conduit (14a) is closed, and in a third switch position, all three conduits (14a-14c) are closed.

4. Method for performing a filling and recirculation process of a blood-side conducting system of a dialysis machine according to claim 3, **characterised in that** the 3-way valve (14) can be activated manually, for which purpose it is equipped with a rotary piston (20) designed with a handle (22).

5. Method for performing a filling and recirculation process of a blood-side conducting system of a dialysis machine according to claim 3 or 4, **characterised in that** the fluid or liquid container (10) is coupled with the arterial blood line section (4) for the filling process based on the single-pass principle by putting the 3-way switch, preferably the 3-way valve (14), of the filling device in the first switch position, and the arterial line section (4) is connected with the venous line section (2) for the circulation process by putting the 3-way switch, preferably the 3-way valve (14), in the second switch position, whereby in this second switch position, the first conduit (14a) of the 3-way switch, preferably the 3-way valve (14), is closed and so the fluid container (10) is fluidically separated from the fluid conducting system of the extracorporeal blood treatment device (10).

6. Method for performing a filling and recirculation process of a blood-side conducting system of a dialysis machine according to claim 5, **characterised in that** in the third switch position of the 3-way switch, preferably the 3-way valve (14), all of its conduits (14a - 14c) are closed so that in this switch position, the two line sections (2, 4) of the fluid conducting system can be disconnected from the fluid container (10).

7. Method for performing a filling and recirculation process of a blood-side conducting system of a dialysis machine according to one of the claims above, **characterised in that** on the fluid blocking mechanism (14), the spike is connected as a connecting device (12) with a Luer-Lock fitting and/or is combined with the fluid blocking mechanism (14) into an integral modular unit.

8. Method for performing a filling and recirculation process of a blood-side conducting system of a dialysis machine according to claim 7, **characterised in that** the spike (12) is connected transition-free without interposition of a bridging line section directly with the fluid blocking mechanism (14).

## Revendications

1. Procédé de réalisation d'un processus de remplissage et de recirculation d'un système de conduite pour le sang d'une machine de dialyse en utilisant un dispositif de remplissage qui a les éléments de construction suivants :
- une pointe (12) qui est adaptée pour le raccordement de l'unique raccord de fluide d'un récipient de fluide médical (10) du système de fluide et
- un dispositif de blocage de fluide (14) actionnable manuellement qui est agencé directement en aval de la pointe (12) et adapté afin de rester relié fluidiquement à la pointe (12) lors du fonctionnement du dispositif de remplissage,
- le dispositif de blocage de fluide (14) présentant au moins un raccord de sortie de fluide en forme de deuxième voie (14b) qui est adapté afin qu'une section de conduite artérielle d'un système de conduite pour le sang puisse être raccordée de manière amovible lors du fonctionnement du dispositif de remplissage à celui-ci et
- le dispositif de blocage de fluide (14) étant un dispositif de séparation à trois voies, de préférence un robinet à trois voies (14), qui est directement agencé en aval de la pointe (12), **caractérisé par** les étapes suivantes :
a. le raccordement de la pointe (12) au seul raccord de fluide du récipient de fluide (10) médical en cas de dispositif de séparation à trois voies (14) bloqué complètement ainsi que le raccordement de la section de conduite artérielle (4) à la deuxième voie (14b) du dispositif de séparation à trois voies (14) encore bloqué en cas de section de conduite de fluide (2) veineuse ouverte vers l'environnement,
b. le rinçage d'une voie d'écoulement de fluide le long de la section de conduite de fluide artérielle (4) et de la section de conduite de fluide veineuse (2) par la liaison fluidique de la pointe (12) à la deuxième voie (14b) du dispositif de séparation à trois voies (14),
c. le blocage complet du dispositif de séparation à trois voies (14) et le raccordement de la section de conduite de fluide veineuse (2) à une troisième voie (14c) du dispositif de séparation à trois voies (14),
d. la recirculation du fluide rempli dans la voie d'écoulement de fluide par court-circuitage de la deuxième et troisième voies (14b, 14c) sur le dispositif de séparation à trois voies en cas de blocage simultané de la pointe (12),
e. le blocage complet du dispositif de séparation à trois voies (14) et le découplage de la section de conduite de fluide (2) veineuse de la troisième voie (14c) ainsi que le nouveau rinçage selon l'étape b. et
f. le blocage complet du dispositif de séparation à trois voies comme préparation pour le raccordement consécutif des sections de conduite de fluide artérielle et veineuse remplies (2, 4) à un patient.

2. Procédé de réalisation d'un processus de remplissage et de recirculation d'un système de conduite pour le sang d'une machine de dialyse selon la revendication 1, **caractérisé en ce qu'**une première voie (14a) du dispositif de séparation à trois voies, de préférence du robinet à trois voies (14) forme l'entrée de fluide qui est couplée à la pointe (12) ou est réalisée comme pointe (12), la section de conduite artérielle (4) pouvant être raccordée à la deuxième voie (14b) du dispositif de séparation à trois voies, de préférence du robinet à trois voies (14) qui forme l'au moins un raccord de sortie, et la section de conduite veineuse (2) du système de conduite de fluide de l'appareil de traitement de sang extracorporel (1) pouvant être raccordée à une troisième voie (14c) du dispositif de séparation à trois voies, de préférence du robinet à trois voies (14).

3. Procédé de réalisation d'un processus de remplissage et de recirculation d'un système de conduite pour le sang d'une machine de dialyse selon la revendication 2, **caractérisé en ce que** le dispositif de séparation à trois voies, de préférence le robinet à trois voies (14) peut être amené ou commuté dans au moins trois positions, parmi lesquelles, dans une première position de commutation, la première voie (14a) est reliée fluidiquement à la deuxième voie (14b) ou permet un écoulement de fluide entre celles-ci, et la troisième voie (14c) est bloquée, dans une deuxième position de commutation la deuxième voie (14b) est reliée fluidiquement à la troisième voie (14c) ou permet un écoulement de fluide entre celles-ci et la première voie (14a) est bloquée et dans une troisième position de commutation, l'ensemble des trois voies (14a-14c) est bloqué.

4. Procédé de réalisation d'un processus de remplissage et de recirculation d'un système de conduite pour le sang d'une machine de dialyse selon la revendication 3, **caractérisé en ce que** le robinet à trois voies (14) peut être actionné manuellement, celui-ci présentant un piston rotatif (20) réalisé avec une manette (22) à cet effet.

5. Procédé de réalisation d'un processus de remplissage et de recirculation d'un système de conduite pour le sang d'une machine de dialyse selon la revendication 3 ou 4, **caractérisé en ce que** le récipient de fluide ou de liquide (10) est couplé pour le processus de remplissage selon le principe à une seule passe à la section de conduite artérielle (4), le dispositif de séparation à trois voies, de préférence le robinet à trois voies (14) du dispositif de remplissage étant amené dans la première position de commutation, et la section de conduite artérielle (4) pour le processus de circulation étant reliée à la section de conduite veineuse (2), le dispositif de séparation à trois voies, de préférence le robinet à trois voies (14) étant amené dans la deuxième position de commutation, dans cette deuxième position de commutation, la première voie (14a) du dispositif de séparation à trois voies, de préférence du robinet à trois voies (14) étant bloquée et ainsi le récipient de liquide (10) étant séparé en fluide du système de conduite de fluide de l'appareil de traitement de sang (10) extracorporel.

6. Procédé de réalisation d'un processus de remplissage et de recirculation d'un système de conduite pour le sang d'une machine de dialyse selon la revendication 5, **caractérisé en ce que** dans la troisième position de commutation du dispositif de séparation à trois voies, de préférence du robinet à trois voies (14), l'ensemble de ses voies (14a-14c) est bloqué de sorte que dans cette position de commutation, les deux sections de conduite (2, 4) du système de conduite de fluide puissent être détachées du récipient de liquide (10).

7. Procédé de réalisation d'un processus de remplissage et de recirculation d'un système de conduite pour le sang d'une machine de dialyse selon l'une quelconque des revendications précédentes, **caractérisé en ce que** la pointe est raccordée au dispositif de blocage de fluide (14) comme moyen de raccordement (12) par un raccord Luer Lock et/ou est réunie avec le dispositif de blocage de fluide (14) en une unité de construction intégrale.

8. Procédé de réalisation d'un processus de remplissage et de recirculation d'un système de conduite pour le sang d'une machine de dialyse selon la revendication 7, **caractérisé en ce que** la pointe (12) est directement reliée sans passage sans intercaler de pièce de conduite chevauchante au dispositif de blocage de fluide (14).
